# FASCICULE DE BREVET EUROPEEN

(11) **EP 2 219 686 B1**
(45) Date de publication et mention de la délivrance du brevet: **11.07.2012**
(21) Numéro de dépôt: 08872023.0
(22) Date de dépôt: 26.11.2008
(51) Int. Cl.: A61L 2/10

(54) **DISPOSITIF DE SÉCURISATION DE LIQUIDE PAR RAYONNEMENTS ULTRAVIOLETS AU POINT D'UTILISATION ET PROCÉDÉ DE SÉCURISATION DE LIQUIDE**
VORRICHTUNG ZUR SICHERUNG EINER FLÜSSIGKEIT DURCH ULTRAVIOLETTSTRAHLUNG AM VERWENDUNGSPUNKT UND VERFAHREN ZUR SICHERUNG EINER FLÜSSIGKEIT
DEVICE FOR MAKING A LIQUID SAFE BY ULTRAVIOLET RADIATION AT THE POINT OF USE AND METHOD FOR MAKING LIQUID SAFE

(30) Priorité: 27.11.2007 FR 0708279
(43) Date de publication de la demande: 25.08.2010
(73) Titulaire: RC-Lux, 38240 Meylan (FR)
(72) Inventeur: PELLET, Xavier, F-38240 Meylan (FR)
(74) Mandataire: Hecké, Gérard
(86) Numéro de dépôt international: PCT/FR2008/001645
(87) Numéro de publication internationale: WO 2009/098394

(56) Documents cités:
- WO-A-00/78681
- WO-A-02/09774
- WO-A-03/031339
- WO-A-2004/073754
- US-A- 3 948 772
- US-A1- 2004 061 069
- US-A1- 2004 140 435

## Description

### Domaine technique de l'invention

L'invention est relative à un dispositif de sécurisation de liquide au point d'utilisation comportant :
- un stérilisateur comprenant une lampe à ultraviolets, un orifice d'entrée et un orifice de sortie dudit liquide,
- des moyens d'alimentation du liquide raccordés à l'orifice d'entrée du stérilisateur,
- des moyens de distribution du liquide, en détente directe, comportant une première extrémité en liaison avec l'orifice de sortie du stérilisateur, et une seconde extrémité conformée en goulot,
- un actionneur relié à une carte électronique pilotant l'allumage de la lampe à ultraviolets et l'ouverture d'une électrovanne pour la distribution du liquide ou la fermeture de ladite électrovanne pour l'arrêt de la distribution du liquide,
- et un capteur de mesure des rayonnements ultraviolets connecté à la carte électronique pour contrôler la fermeture de l'électrovanne lors de l'émission par le capteur de mesure d'un signal correspondant à une quantité d'ultraviolets inférieure à un seuil prédéterminé,
- ladite la carte électronique comporte des moyens pour contrôler l'ouverture de l'électrovanne lorsque le capteur mesure une quantité d'ultraviolets supérieure au seuil prédéterminé pour un débit donné, et
- le dispositif est équipé de moyens d'ajustement de débit adaptés au seuil prédéterminé.

### État de la technique

Il existe des dispositifs de sécurisation de liquide par rayonnements ultraviolets traitant le liquide au point d'utilisation (généralement un goulot où le liquide est récupéré) de manière à sécuriser ce dernier selon une norme.

On définit par liquide sécurisé un liquide ayant été irradié par une quantité de rayonnements ultraviolets suffisante pour éradiquer des micro-organismes présents dans le liquide. La longueur d'onde optimale pour éradiquer les micro-organismes (virus, bactéries, algues, etc.) correspond aux ultraviolets-C (254nm) qui sont capables de pénétrer au coeur de l'ADN pour perturber le métabolisme des cellules jusqu'à leur destruction. Un seuil d'ultraviolets correspond à une quantité d'énergie UV cumulée, fonction de la puissance du rayonnement et du temps d'irradiation, que doivent absorber les micro-organismes vivants pour être détruits.

À titre d'exemple, le tableau suivant présente les quantités, exprimées en millijoules/cm², nécessaire pour éradiquer 99,9% des micro-organismes :

| **Bactéries** | **Quantité** |
|---|---|
| Bacillus Anthracis | 8,5 mJ/cm² |
| E.Coli | 10,5 mJ/cm² |
| Legionella Pneumophilia | 6,9 mJ/cm² |
| Pseudomonas aeruginosa | 10 mJ/cm² |
| Salmonella enteridis | 9 mJ/cm² |
| Streptococcus Faecalis | 10 mJ/cm² |

| **Algues** | |
|---|---|
| Chlorella Vulgaris | 22 mJ/cm² |

| **Protozoaires** | |
|---|---|
| Cryptosporidium | 16 mJ/cm² |

| **Virus** | |
|---|---|
| Hepatitis | 8 mJ/cm² |

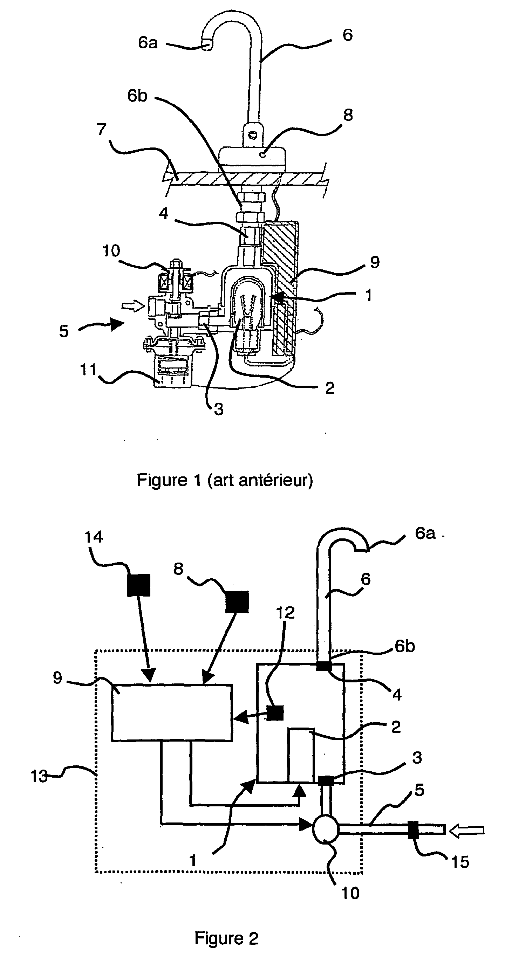

Le document US-B2-6909101 décrit un tel dispositif comme illustré à la figure 1. Le dispositif de traitement est un appareil de purification de l'eau comportant un stérilisateur 1 comprenant une lampe à ultraviolets 2, un orifice d'entrée 3 et de sortie 4 d'un liquide pouvant être de l'eau. L'orifice d'entrée 3 est raccordé à des moyens d'alimentation 5 en eau. L'orifice de sortie 4 est relié à une première extrémité 6b de moyens de distribution 6 de l'eau. Une seconde extrémité des moyens de distribution 6 est conformée en goulot 6a servant de point d'utilisation. Lorsque le dispositif est installé sur un support 7, le stérilisateur 1 et les moyens d'alimentation 5 se trouvent en dessous de ce support 7 et les moyens de distribution 6 traversent le support 7. Un actionneur 8 se situe au niveau de la jonction entre une partie supérieure du support 7 et les moyens de distribution 6. Un tel actionneur 8 peut être un capteur infrarouge. L'actionneur 8 est relié à une carte électronique 9 pilotant l'ouverture d'une électrovanne 10 se situant en amont du stérilisateur 1 par rapport au sens d'écoulement de l'eau (illustré par une flèche sur la figure 1) l'amont étant tout ce qui se trouve avant l'orifice d'entrée 3 du stérilisateur et l'aval tout ce qui se trouve après l'orifice de sortie 4 du stérilisateur. Un détecteur de pression 11 est placé entre l'électrovanne 10 et l'orifice d'entrée 3 de l'eau du stérilisateur 1. Lorsque le détecteur de pression 11 détecte une pression supérieure à un certain seuil, le détecteur de pression 11 en informe la carte électronique 9 qui allume la lampe 2 à ultraviolets du stérilisateur 1. Ainsi, la lampe est allumée uniquement lorsqu'il est sûr que cette dernière recevra de l'eau afin d'éviter son endommagement. L'eau traversant le stérilisateur est traitée par irradiation des rayonnements ultraviolets émis par la lampe 2. Un tel dispositif ne permet pas de garantir que chaque goutte d'eau, sortant du goulot 6a des moyens de distribution 6, a été sécurisée lors de son passage dans le stérilisateur 1. Un tel dispositif sera, en particulier, utilisé dans un mode de fonctionnement intermittent tel un robinet de cuisine. Le temps d'allumage de la lampe 2 n'étant pas instantané, quelques secondes à quelques minutes pour atteindre une quantité de rayonnements ultraviolets suffisants, l'eau distribuée n'est pas sécurisée durant ce laps de temps.

Le document US5611918 décrit un dispositif de traitement de l'eau limitant le problème du dispositif précédent. Le dispositif de traitement comporte un stérilisateur comportant un orifice d'entrée et de sortie du liquide, un détecteur de flux d'eau, placé en amont du stérilisateur par rapport au sens d'écoulement de l'eau, relié à une carte électronique et une lampe à ultraviolets aussi reliée à la carte électronique. La carte électronique comporte deux modes de fonctionnement, lorsque aucun flux d'eau est détecté par le détecteur de flux, la lampe est alimentée par la carte électronique suivant une faible intensité. A contrario, lorsqu'un flux d'eau est détecté, la lampe est alimentée par une forte intensité. À faible intensité, les filaments de la lampe restent faiblement excités ce qui permet leur ré-intensification rapide lorsqu'un flux d'eau est détecté. Ce dispositif bien que limitant le nombre de gouttes d'eau non sécurisées ne permet pas de garantir que toutes les gouttes du liquide traversant le dispositif soient sécurisées. De plus, ce dispositif génère des pertes d'énergie conséquentes. Il est difficile d'installer ce dispositif au niveau d'un point d'utilisation d'eau potable. Dans ce dernier cas, il est nécessaire que le stérilisateur fonctionne de manière intermittente, c'est-à-dire que quand le liquide n'est plus distribué, la lampe à rayonnement ultraviolets doit s'éteindre. En effet, l'eau contient des nitrates, lorsque le seuil de rayonnements ultraviolets dépasse 400mJ/cm², les nitrates se transforment en nitrites dangereux pour la santé. Cette transformation des nitrates en nitrites peut avoir lieu même si la lampe est allumée à faible intensité. De plus, si la lampe reste allumée sans que le liquide ne soit distribué, cela augmente la température du liquide et favorise le développement de biofilms dans les moyens de distribution sans parler du fait qu'une eau chaude n'est pas agréable à consommer.

Le document WO2004/073754 décrit un dispositif de stérilisation d'un fluide au point d'utilisation pour une douche. Le dispositif comporte un stérilisateur muni d'une lampe à rayonnements ultraviolets et un capteur photosensible mesurant la quantité d'ultraviolets émise par la lampe. La distribution du liquide est asservie à un délai de deux minutes nécessaire à l'allumage de la lampe, passé ce délai la distribution peut commencer. En fonctionnement, si le capteur mesure une quantité d'ultraviolets inférieure à un certain seuil, la distribution est stoppée. Un tel dispositif est viable dans le domaine des douches mais ne peut servir dans le domaine des fontaines à eau potable à fonctionnement intermittent, la temporisation de deux minutes ne pouvant être tolérée par un utilisateur souhaitant se désaltérer.

### Objet de l'invention

L'objet de l'invention consiste à réaliser un dispositif de sécurisation de liquide au point d'utilisation par rayonnements ultraviolets garantissant une distribution de liquide totalement sécurisée dans un mode de fonctionnement intermittent.

Ce but est atteint par le fait que la carte électronique comporte des moyens pour contrôler l'ouverture de l'électrovanne lorsque le capteur mesure une quantité d'ultraviolets supérieure au seuil prédéterminé pour un débit donné, et en ce que le dispositif est équipé de moyens d'ajustement de débit adaptés au seuil prédéterminé.

Un tel dispositif garantit que chaque goutte du liquide traversant le stérilisateur soit traitée par une quantité suffisante de rayonnements ultraviolets pour garantir une distribution de liquide sécurisé.

Selon une variante, électrovanne est située en amont du stérilisateur par rapport au sens d'écoulement du liquide.

Selon une caractéristique de l'invention, les moyens de distribution du liquide comportent une tubulure laissée libre, et sans obstacle, en écoulement de l'orifice de sortie du stérilisateur au goulot des moyens de distribution.

Selon un développement, les moyens de distribution présentent une surface interne en contact avec le liquide, réalisée en matériaux biocides, notamment dopée en argent, ou réfléchissante des rayonnements ultraviolets émis par la lampe.

Selon une autre caractéristique de l'invention, des moyens d'accélération de l'allumage de la lampe à ultraviolet optimisent la vitesse de distribution du liquide.

Selon une variante, la carte électronique comporte des moyens de déclenchement auxiliaire pour purger le stérilisateur, les moyens de distribution et les moyens d'alimentation.

L'invention concerne aussi un procédé d'utilisation du dispositif, caractérisé par les étapes successives suivantes :
- on émet un premier signal vers une carte électronique en activant un actionneur,
- on pilote l'allumage d'une lampe à rayonnements ultraviolets d'un stérilisateur lorsque la carte électronique reçoit le premier signal,
- on mesure une quantité de rayonnements ultraviolets émis par la lampe par l'intermédiaire d'un capteur de mesure émettant un second signal vers la carte électronique jusqu'à dépassement d'un seuil prédéterminé pour un débit donné,
- on ouvre l'électrovanne à la réception d'un troisième signal émis par la carte électronique lorsque ladite carte reçoit le second signal dépassant le seuil prédéterminé,

et on ferme l'électrovanne si la quantité de rayonnements ultraviolets émis par la lampe repasse en dessous dudit seuil, ou lorsque l'actionneur est à nouveau activé.

### Description sommaire des dessins

D'autres avantages et caractéristiques ressortiront plus clairement de la description qui va suivre d'un mode particulier de réalisation de l'invention donnés à titre d'exemples non limitatif et représenté aux dessins annexés, dans lesquels :
La figure 1 illustre une vue en coupe d'un dispositif selon l'art antérieur.
La figure 2 illustre schématiquement un mode de réalisation de l'invention.

### Description d'un mode préférentiel de réalisation

Le dispositif de sécurisation de liquide au point d'utilisation, illustré à la figure 2, comporte un stérilisateur 1 du liquide comprenant une lampe 2 à ultraviolets et un capteur 12 de mesure des rayonnements ultraviolets émis par la lampe 2. Le capteur est, de préférence, disposé dans le stérilisateur en regard de la lampe 2 à rayonnements ultraviolets. Le stérilisateur 1 comporte en plus un orifice d'entrée 3 du liquide, auquel est raccordé des moyens d'alimentation 5, et un orifice de sortie 4 en liaison à une première extrémité 6b de moyens de distribution 6. Les moyens de distribution 6 comportent une seconde extrémité conformée en un goulot 6a correspondant au point d'utilisation. Les moyens de distribution 6 sont en détente directe, c'est-à-dire que le débit du liquide entre l'orifice d'entrée 3 et les moyens de distribution 6 est le même. Le liquide n'est pas stocké dans un ballon, par exemple, où il pourrait se contaminer. Le dispositif comporte une carte électronique 9 sur laquelle sont connectés un actionneur 8 et le capteur de mesure 12 des rayonnements ultraviolets. La carte électronique 9 pilote une électrovanne 10 et l'allumage de la lampe 2 du stérilisateur 1 en fonction des données de l'actionneur et du capteur. L'électrovanne est, de préférence, située en amont du stérilisateur 1 par rapport au sens d'écoulement du liquide (indiqué par une flèche sur la figure 2) et permet la distribution du liquide par ouverture de l'électrovanne 10 ou l'arrêt de la distribution par fermeture de l'électrovanne 10 sur activation de l'actionneur. La carte électronique 9 comporte des moyens pour contrôler l'ouverture de l'électrovanne 10 lorsque le capteur 12 mesure une quantité d'ultraviolets supérieure à un seuil prédéterminé pour un débit de liquide donné.

Lorsque l'actionneur 8 est activé, un signal est transmis à la carte électronique 9, celle-ci détermine que le liquide doit être distribué. La carte électronique 9 pilote alors l'allumage de la lampe 2 à ultraviolets. Par l'intermédiaire du capteur 12, la carte électronique 9 reçoit des signaux correspondant à la quantité mesurée des rayonnements à ultraviolets émis par la lampe 2. Lorsque la mesure des rayonnements considérés est supérieure à un certain seuil prédéterminé, la carte électronique 9 détermine que le stérilisateur 1 est prêt pour traiter le liquide. À ce moment, la carte électronique 9 pilote l'ouverture de l'électrovanne 10, et le liquide traverse le stérilisateur 1 par l'intermédiaire de l'orifice d'entrée 3. Le liquide est ensuite irradié, par les rayonnements ultraviolets émis par la lampe 2, chaque goutte du liquide ressort traitée par l'orifice de sortie 4. Pendant la distribution de liquide, si la quantité de rayonnements ultraviolets mesurée par le capteur repasse en dessous du seuil prédéterminé, la carte électronique 9 pilote la fermeture de la vanne 10 afin d'arrêter la distribution du liquide.

Le seuil prédéterminé de la quantité de rayonnements ultraviolets est défini pour un débit de liquide donné et correspond à des normes sanitaires et/ou médicales permettant de détruire des micro-organismes (à titre d'exemple voir le tableau précité). Afin d'éviter tout développement de micro-organisme dans les moyens de distribution 6 du liquide, ceux-ci peuvent comporter une tubulure laissée libre, et sans obstacle, en écoulement de l'orifice de sortie 4 du stérilisateur au goulot 6a des moyens de distribution 6.

Le seuil prédéterminé dépend du débit du liquide traversant le stérilisateur. Afin d'installer le dispositif de sécurisation de liquide sur n'importe quelle installation et/ou de pouvoir pallier aux fluctuations de débit, dues à des variations de pression, de l'installation où est utilisé le dispositif, ce dernier est muni de moyens d'ajustement de débit 15 adaptés au seuil. Les moyens d'ajustement de débit 15 peuvent se présenter sous la forme d'un diaphragme ou d'une électrovanne autolimitante. Ainsi, il est possible de garantir un débit dans le stérilisateur correspondant au seuil prédéterminé de rayonnements ultraviolets. Une électrovanne autolimitante peut comporter un fluxmètre pilotant l'ouverture et la fermeture de l'électrovanne autolimitante pour assurer un débit constant. Selon une variante, il est aussi possible de modifier le seuil prédéterminé correspondant à une quantité de rayonnements ultraviolets en fonction du débit. Plus le débit sera faible plus le seuil correspondant sera diminué. Les moyens d'ajustement de débit 15 peuvent à titre d'exemple être une simple plaque, un diaphragme ou une électrovanne autolimitante reliés à la carte électronique 9. La carte électronique 9 contrôle alors le débit et calcule le seuil associé. Les moyens d'ajustement de débit 15 permettent d'installer le dispositif sans réglage préalable du débit, le tout étant automatisé.

Le procédé de sécurisation de liquide au point d'utilisation faisant usage d'un stérilisateur à rayonnements ultraviolets et d'une électrovanne tels que décrits ci-dessus, comporte les étapes successives suivantes :
- on émet un premier signal vers une carte électronique 9 en activant un actionneur 8,
- on pilote l'allumage d'une lampe 2 à rayonnements ultraviolets d'un stérilisateur 1 lorsque la carte électronique 9 reçoit le premier signal,
- on mesure une quantité de rayonnements ultraviolets émis par la lampe 2 par l'intermédiaire d'un capteur 12 de mesure émettant un second signal vers la carte électronique 9 jusqu'à dépassement d'un seuil prédéterminé garantissant qu'un liquide traversant le stérilisateur sera sécurisé pour un débit donné,
- on ouvre l'électrovanne à la réception d'un troisième signal émis par la carte électronique 9 lorsque ladite carte 9 reçoit le second signal dépassant le seuil prédéterminé.

Une fois l'électrovanne ouverte, le liquide est distribué au goulot 6a du dispositif, ce liquide est totalement sécurisé, chaque goutte a été traité par une quantité de rayonnements ultraviolets correspondant aux normes sanitaires. L'électrovanne 10 est refermée si la quantité de rayonnements ultraviolets émis par la lampe repasse en dessous dudit seuil (défaut de lampe, entartrage, changement de la nature de l'eau, etc.), ou lorsque l'actionneur est à nouveau activé pour demander l'arrêt de la distribution, sécurisant ainsi le liquide durant toute sa distribution.

De préférence, lorsque l'actionneur a été activé pour demander l'arrêt de la distribution du liquide, l'électrovanne est fermée puis la lampe 2 à rayonnements ultraviolets est éteinte après un délai suivant la fermeture complète de l'électrovanne 10 par exemple par temporisation de quelques secondes permettant au liquide contenu dans le stérilisateur d'être suffisamment traité afin de ne pas polluer le stérilisateur. Ce délai est déterminé par calcul en utilisant des méthodes conventionnelles pour déterminer une irradiation d'un liquide suffisante en fonction du seuil prédéterminé, de la puissance de la lampe à ultraviolets et du volume de liquide que peut accueillir le stérilisateur.

Les rayonnements ultraviolets n'ayant pas d'effets rémanents (à l'opposé du chlore), le liquide non sécurisé peut contaminer les moyens de distribution 6 et favoriser le développement d'un biofilm (agrégation de micro-organismes) en aval du stérilisateur ce qui rendrait impossible toute sécurisation du liquide au goulot 6a. Tout obstacle a donc intérêt à être placé en amont du stérilisateur 1, ainsi l'électrovanne 10, les moyens d'ajustement de débit 15 sont, de préférence placés en amont du stérilisateur.

De plus, afin de limiter le développement de biofilms dans les moyens de distribution 6, ceux-ci présentent une surface interne en contact avec le liquide la plus lisse possible. La surface en contact avec le liquide est en matériaux biocides, par exemple dopée en argent. Un tel dopage limite la formation de biofilms. Les rayonnements ultraviolets peuvent aussi être amenés dans les moyens de distribution 6 par des fibres optiques, pour chaque fibres une extrémité est orientée vers la lampe 2 à ultraviolets dans le stérilisateur et l'autre extrémité débouche dans les moyens de distribution 6. Selon un développement, la surface interne est réfléchissante aux rayonnements ultraviolets émis par la lampe 2 à ultraviolets, permettant leur propagation dans les moyens de distribution.

Dans une variante, la lampe 2 du stérilisateur peut être constituée par une lampe à ultraviolets sans électrode pour permettre un nombre de cycle d'allumage/extinction illimité. Une telle lampe évite les désagréments qui peuvent survenir lors de l'utilisation d'une lampe à électrodes. En effet un grand nombre de cycle d'allumage/extinction endommage les lampes à électrodes, provoquant de nombreuses interventions de maintenance. À titre d'exemple, la lampe utilisée pour traiter le liquide par irradiation aux rayonnements ultraviolets peut être à diodes, à induction, micro-ondes etc. Bien entendu des lampes à électrodes peuvent être utilisées mais devront être remplacées plus souvent, de plus de telle lampes mettent plus de temps pour atteindre la quantité de rayonnements ultraviolets suffisant pour sécuriser le liquide.

Lorsque le dispositif n'est pas utilisé, la lampe 2 peut être allumée régulièrement pendant un court instant de manière temporisée pour réaliser une barrière bactériologique entre l'amont et l'aval du stérilisateur sans que l'électrovanne soit ouverte. Cette barrière permet d'augmenter la sécurisation du liquide lorsque l'électrovanne est située en amont du stérilisateur. En effet, même lorsque l'eau ne coule pas, les bactéries sont mobiles. La barrière permet d'éviter que des bactéries migrent de l'amont vers l'aval du stérilisateur et/ou inversement. La barrière bactériologique évite, lorsque l'électrovanne 10 est fermée, une rétrocontamination et permet d'éviter le développement de bactéries dans le stérilisateur. Dans une variante, la carte électronique 9 peut comporter des moyens de déclenchement auxiliaire de purge automatique pour purger le stérilisateur 1, les moyens de distribution 6 et les moyens d'alimentation 5. Lorsque le dispositif n'est pas utilisé depuis un certain temps, qui peut être paramétrable, les moyens de déclenchement auxiliaire de la carte électronique 9 exécutent un cycle de purge du système. Ce cycle est semblable à une utilisation du dispositif par activation de l'actionneur 8 tel que décrit ci-dessus. Pour déterminer si un cycle de purge doit être réalisé, la carte électronique 9 garde en mémoire le moment où a été utilisé le dispositif pour la dernière fois, si le temps écoulé est supérieur à un certain seuil, alors la carte électronique 9 initie l'allumage de la lampe 2. Lorsque le capteur de mesure informe la carte électronique 9 que la lampe émet une quantité de rayonnements d'ultraviolets suffisante pour garantir la distribution d'un liquide sécurisé, la carte électronique 9 pilote l'ouverture de l'électrovanne 10 pour effectuer une purge par rinçage avec du liquide sécurisé, puis referme l'électrovanne une fois l'opération de purge effectuée. Dans une autre variante, la purge est réalisée en deux étapes. Sur commande de la carte électronique 9, lorsque la lampe 2 émet suffisamment de rayonnements ultraviolets il est adjoint, automatiquement ou sur demande, un produit détartrant et antibiofilm contenu dans une réserve reliée au stérilisateur, puis le rinçage est effectué avec du liquide sécurisé aux ultraviolets. Le produit antibiofilm permet de supprimer des micro-organismes (bactéries, champignons, algues, ou protozaires) ayant adhérés entre eux pour former un biofilm sur une surface intérieure, d'écoulement du liquide, du stérilisateur 1 et des moyens de distribution 6.

Les moyens d'alimentation 5 en liquide peuvent comporter un filtre de type filtre à charbon (non représenté) permettant d'améliorer le goût et de réduire la présence de chlore dans le liquide. Le filtre à charbon favorisant l'apparition de micro-organismes, il sera, de préférence, situé en amont du stérilisateur. Un cycle de purge permettra de renouveler l'eau présente dans le filtre afin d'éviter la prolifération de micro-organismes en amont du stérilisateur 1.

Selon une caractéristique de l'invention, des moyens d'accélération de l'allumage de la lampe 2 à ultraviolet optimisent la vitesse de distribution du liquide. La vitesse de distribution peut être définie par le temps d'attente d'un utilisateur entre l'activation de l'actionneur 8 et le moment où le liquide sécurisé par irradiation aux rayonnements ultraviolets sort des moyens de distribution 6 par le goulot 6a. Les moyens d'accélération peuvent correspondrent à la puissance fournie à la lampe 2. La carte électronique 9 comporte un premier mode d'alimentation de la lampe 2 doté d'une puissance supérieure à la puissance de référence pour irradier le liquide et un deuxième mode d'alimentation de la lampe 2 ayant une puissance régulée à la puissance de référence. La puissance de référence correspond à la puissance d'alimentation requise pour que la lampe 2 émette des rayonnements ultraviolets correspondant au seuil prédéterminé. La lampe 2 est donc suralimentée jusqu'à ce que la mesure effectuée par le capteur 12 atteigne le seuil d'ultraviolets désiré, au moment où le seuil est atteint, la puissance est régulée par la carte électronique 9 pour maintenir une quantité d'irradiation constante.

Dans une autre variante, les moyens d'accélération de la distribution du liquide correspondent à une anticipation de l'allumage de la lampe 2 par des flashs réguliers qui entretiennent la sécurité de l'eau dans le stérilisateur lorsque l'eau ne coule pas, ou par un détecteur de présence (non représenté). Le détecteur est alors relié à la carte électronique 9 pour piloter l'allumage de la lampe lorsqu'une présence est détectée par le détecteur. Ainsi lorsqu'un utilisateur active l'actionneur, le temps de distribution de liquide est inférieur au temps de chauffe de la lampe 2 voir instantané si la quantité cumulée de rayonnements ultraviolets de la lampe a été suffisante. La lampe 2 pouvant ensuite être éteinte lorsque le détecteur ne détecte plus de mouvements après une certaine temporisation. Cette variante peut coopérer avec l'une des deux ou les deux variantes décrites ci-dessus.

Selon une autre variante, il est possible d'utiliser les moyens d'ajustement de débit 15 couplés aux moyens d'accélération de l'allumage de la lampe, afin de permettre un débit limité de liquide sécurisé pendant le temps de chauffe de la lampe 2 puis un débit nominal lorsque la lampe 2 émet la quantité de rayonnements ultraviolets associée au débit nominal.

Le stérilisateur 1, l'électrovanne 10, la carte électronique 9 peuvent être intégrés dans un boîtier 13 étanche et reliés à une alimentation commune. Si le dispositif comporte des moyens d'ajustement de débit 15, ces derniers peuvent aussi être intégré au boîtier. De préférence, le boîtier a un indice de protection 23 (IP23). L'indication IP23 est une norme de sécurité, elle signifie pour son premier chiffre (2) que le boîtier est protégé contre l'introduction de corps solides supérieurs à 12 mm et pour son deuxième chiffre (3) que le boîtier est aussi protégé contre de la pluie tombant sur le boîtier avec un angle de 60°. De tels boîtiers peuvent être utilisés sans danger près de points d'eau d'une salle de bain ou d'une cuisine. Dans une variante, le boîtier comporte une trappe d'accès au stérilisateur permettant de changer la lampe sans avoir à démonter le boîtier, facilitant ainsi les opérations de maintenance du dispositif.

La carte électronique 9 peut comporter au moins une interface réseau (non représentée) connectée à un réseau informatique. Cette interface est de type filaire ou réseau sans fil, et permet de centraliser les informations d'un stérilisateur et notamment de pouvoir effectuer un diagnostic. A titre d'exemple, il est possible de connaître l'état de la lampe, de réaliser des tests, d'effectuer une purge, etc. La carte électronique 9 pourra être pilotée à distance de manière à simuler une activation manuelle de l'actionneur.

Le dispositif peut incorporer des moyens d'affichage 14 pour signaler différents états du stérilisateur. Ces moyens d'affichage se situent au niveau du boîtier 13 ou peuvent être déportés au niveau d'un évier sur lequel le dispositif est installé, permettant d'informer l'utilisateur de l'état du stérilisateur (qualité du liquide, préchauffage de la lampe, etc.).

Les informations de maintenance du système peuvent être aussi communiquées à l'utilisateur. Un besoin de nettoyage dû à l'encrassement ou l'entartrage du système peut, par exemple, être détecté par la carte électronique et le capteur de mesure UV en comparant la pente standard de vieillissement de la lampe et la pente réelle mesurée par le capteur.

L'actionneur 8 est un actionneur manuel permettant la mise en service ou l'arrêt de la distribution de l'eau. Il peut, à titre d'exemple, se présenter sous la forme d'un interrupteur, d'un détecteur infrarouge, d'un détecteur de présence, d'un système de reconnaissance vocal, ou d'un robinet électronique ne comportant pas de vanne dont l'aspect extérieur est celui d'un robinet traditionnel, etc. Afin que son utilisation ne déroute pas un utilisateur, l'actionneur 8 peut être agencé sur une embase des moyens de distribution 6, en aval du stérilisateur. L'actionneur peut comporter une rondelle compatible avec les trous standard des éviers et, dans le cas où celui-ci correspondrait d'aspect extérieur à un robinet traditionnel, un potentiomètre relié à la carte électronique 9. Un robinet électronique à potentiomètre peut contrôler le débit du liquide ou un mélangeur situé en amont de l'électrovanne 10 afin de régler la température du liquide. L'actionneur peut aussi incorporer les moyens d'affichage pour signaler différents états du système à un utilisateur.

La tubulure formant les moyens de distribution 6 peut se présenter sous la forme d'un col-de-cygne amovible permettant de faciliter le nettoyage si besoin.

Le dispositif et ses variantes telles que décrites ci-dessus peut être installé au niveau d'un évier, pour permettre la distribution de liquide et plus particulièrement d'eau. Ce dispositif peut notamment être utilisé dans les hôpitaux où les besoins en eau sécurisée sont importants, ainsi que dans les pays où le réseau d'eau local est peu fiable. Le dispositif permet de garantir que chaque goutte de liquide passant par le stérilisateur est irradiée par une quantité calibrée de rayonnements ultraviolets.

## Revendications

1. Dispositif de sécurisation de liquide au point d'utilisation comportant :
- un stérilisateur (1) comprenant une lampe (2) à ultraviolets, un orifice d'entrée (3) et un orifice de sortie (4) dudit liquide,
- des moyens d'alimentation (5) du liquide raccordés à l'orifice d'entrée (3) du stérilisateur,
- des moyens de distribution (6) du liquide, en détente directe, comportant une première extrémité (6b) en liaison avec l'orifice de sortie (4) du stérilisateur, et une seconde extrémité conformée en goulot (6a),
- un actionneur (8) relié à une carte électronique (9) pilotant l'allumage de la lampe (2) à ultraviolets et l'ouverture d'une électrovanne (10) pour la distribution du liquide ou la fermeture de ladite électrovanne pour l'arrêt de la distribution du liquide,
- et un capteur (12) de mesure des rayonnements ultraviolets connecté à la carte électronique (9) pour contrôler la fermeture de l'électrovanne (10) lors de l'émission par ledit capteur (12) d'un signal correspondant à une quantité d'ultraviolets inférieure à un seuil prédéterminé,
**caractérisé en ce que** ladite la carte électronique (9) comporte des moyens pour contrôler l'ouverture de l'électrovanne (10) lorsque le capteur (12) mesure une quantité d'ultraviolets supérieure au seuil prédéterminé pour un débit donné, et **en ce que** le dispositif est équipé de moyens d'ajustement de débit (15) adaptés au seuil prédéterminé.

2. Dispositif selon la revendication 1, **caractérisé en ce que** ladite électrovanne (10) est située en amont du stérilisateur (1) par rapport au sens d'écoulement du liquide.

3. Dispositif selon l'une des revendications 1 et 2, **caractérisé en ce que** les moyens de distribution (6) du liquide comportent une tubulure laissée libre, et sans obstacle, en écoulement de l'orifice de sortie (4) du stérilisateur (1) jusqu'au goulot (6a) des moyens de distribution (6).

4. Dispositif selon la revendication 3, **caractérisé en ce que** les moyens de distribution (6) comportent une surface interne, en contact avec le liquide, et réalisée en matériaux biocides, notamment dopée en argent, et/ou réfléchissante aux rayonnements ultraviolets émis par la lampe (2) à ultraviolets.

5. Dispositif selon l'une quelconque des revendications 1 à 4, **caractérisé en ce qu'**il comporte des moyens d'accélération de l'allumage de la lampe (2) à ultraviolets.

6. Dispositif selon la revendication 5, **caractérisé en ce que** la carte électronique (9) est reliée à un détecteur de présence, pour anticiper l'allumage de la lampe à ultraviolets lorsqu' une présence est détectée par ledit détecteur.

7. Dispositif selon l'une quelconque des revendications 1 à 6, **caractérisé en ce que** la carte électronique (9) comporte des moyens de déclenchement auxiliaire pour purger le stérilisateur (1), les moyens de distribution (6) et les moyens d'alimentation (5) du liquide.

8. Dispositif selon l'une quelconque des revendications 1 à 7, **caractérisé en ce que** l'électrovanne (10) étant fermée, le dispositif comporte une barrière bactériologique entre l'amont et l'aval du stérilisateur, ladite barrière bactériologique étant générée par l'allumage régulier de la lampe (2) à rayonnements ultraviolets.

9. Procédé de sécurisation de liquide au point d'utilisation faisant usage d'un dispositif comportant un stérilisateur à rayonnements ultraviolets et d'une électrovanne selon l'une quelconque des revendications 1 à 8, **caractérisé par** les étapes successives suivantes :
- on émet un premier signal vers une carte électronique (9) en activant un actionneur (8),
- on pilote l'allumage d'une lampe (2) à rayonnements ultraviolets d'un stérilisateur (1) lorsque la carte électronique (9) reçoit le premier signal,
- on mesure une quantité de rayonnements ultraviolets émis par la lampe (2) à ultraviolets par l'intermédiaire d'un capteur de mesure (12) émettant un second signal vers la carte électronique (9) jusqu'à dépassement d'un seuil prédéterminé pour un débit donné,
- on ouvre l'électrovanne (10) à la réception d'un troisième signal émis par la carte électronique (9) lorsque ladite carte (9) reçoit le second signal dépassant le seuil prédéterminé,
- et on ferme l'électrovanne (10) si la quantité de rayonnements ultraviolets émis par la lampe à ultraviolets repasse en dessous dudit seuil, ou lorsque l'actionneur est à nouveau activé.

10. Procédé selon la revendication 9, **caractérisé en ce que** l'actionneur étant à nouveau activé pour demander l'arrêt de la distribution de liquide, l'électrovanne (10) est fermée et ladite lampe (2) à rayonnements ultraviolets est éteinte après un délai fonction du seuil prédéterminé, de la puissance de la lampe à ultraviolets (2) et du volume du stérilisateur.

## Claims

1. Device for making a liquid safe at the dispensing point comprising:
- a sterilizer (1) comprising a UV lamp (2), an input opening (3) and an output opening (4) for said liquid,
- liquid supplying means (5) connected to the input opening (3) of the sterilizer,
- liquid distribution means (6), with a direct expansion, comprising a first end (6b) in connection with the output opening (4) of the sterilizer, and a second neck-shaped end (6a),
- an actuator (8) connected to an electronic board (9) controlling the switching-on of the UV lamp (2) and the opening of an electromagnetic valve (10) for the liquid distribution or the closing of said electromagnetic valve for stopping the liquid distribution,
- and a UV measurement sensor (12) connected to the electronic board (9) for controlling the closing of the electromagnetic valve (10) during the emission, by said sensor (12), of a signal corresponding to a quantity of ultraviolet radiations lower than a preset threshold, **characterized in that** said electronic board (9) comprises means for controlling the opening of the electromagnetic valve (10) when the sensor (12) measures a quantity of ultraviolet radiations higher than the preset threshold for a given flow rate, and **in that** the device is provided with flow rate adjustment means (15) adapted to the preset threshold.

2. Device according to claim 1, **characterized in that** said electromagnetic valve (10) is located on the upstream side of the sterilizer (1) by reference of the direction of flow of the liquid.

3. Device according to anyone of the claims 1 and 2, **characterized in that** the liquid distribution means (6) comprise a nozzle allowing a free flow, without any obstacle, from the output opening (4) of the sterilizer (1) to the neck (6a) of the distribution means (6).

4. Device according to claim 3, **characterized in that** the distribution means (6) comprise an internal surface in contact with the liquid and made out of biocide materials, in particular doped with silver, and/or an internal surface reflective for ultraviolet radiations emitted by the UV lamp (2).

5. Device according to anyone of the claims 1 to 4, **characterized in that** it comprises of acceleration means for the switching on of the UV lamp (2).

6. Device according to claim 5, **characterized in that** the electronic board (9) is connected to a presence detector for anticipating the switching-on of the UV lamp when a presence is detected by said detector.

7. Device according to anyone of the claims 1 to 6, **characterized in that** the electronic board (9) comprises auxiliary triggering means for purging the sterilizer (1), the liquid distribution means (6) and the liquid supplying means (5).

8. Device according to anyone of the claims 1 to 7, **characterized in that**, as the electromagnetic valve (10) is closed, the device comprises a bacteriological barrier between the upstream side and the downstream side of the sterilizer, said bacteriological barrier being generated by the regular switching-on of the UV lamp (2).

9. Method for making a liquid safe at the dispensing point, said method using a device comprising a UV sterilizer and an electromagnetic valve according to anyone of the claims 1 to 8, **characterized by** the following successive steps:
- a first signal is emitted towards an electronic board (9) by activating an actuator (8),
- the switching-on of a UV lamp (2) of a sterilizer (1) is controlled when the electronic board (9) receives the first signal,
- a quantity of ultraviolet radiations emitted by the UV lamp (2) is measured by means of a measurement sensor (12) emitting a second signal towards the electronic board (9) until a preset threshold is exceeded for a given flow rate,
- the electromagnetic valve (10) is opened when receiving a third signal emitted by the electronic board (9) when said board (9) receives the second signal exceeding the preset threshold,
- the electromagnetic valve (10) is closed when the quantity of ultraviolet radiations emitted by the UV lamp goes back below said threshold, or when the actuator is activated again.

10. Method according to claim 9, **characterized in that**, when the actuator is activated again for requiring the stop of the liquid distribution, the electromagnetic valve (10) is closed and said UV lamp (2) is switched off after a duration depending on the preset threshold, the power of the UV lamp (2) and the volume of the sterilizer.

## Patentansprüche

1. Vorrichtung zum Schützen einer Flüssigkeit am Ort der Benutzung, die umfasst:
- einen Sterilisator (1), der eine UV-Lampe (2), eine Eintrittsöffnung (3) und eine Austrittsöffnung (4) für die genannte Flüssigkeit umfasst,
- Mittel zum Zuführen (5) der Flüssigkeit, die an die Eintrittsöffnung (3) des Sterilisators angeschlossen sind,
- Mittel zum Abgeben (6) der Flüssigkeit, in direkter Verteilung, die ein erstes Ende (6b), das in Verbindung mit der Austrittsöffnung (4) des Sterilisators steht, und ein zweites Ende umfasst, das in Form eines engen Halses (6a) vorgesehen ist,
- eine Betätigungseinrichtung (8), die mit einer Magnetkarte (9) verbunden ist, die das Einschalten der UV-Lampe (2) und das Öffnen des Magnetventils (10) zur Abgabe der Flüssigkeit oder das Schließen des genannten Magnetventils zum Stoppen der Abgabe der Flüssigkeit steuert,
- sowie einen Sensor (12) zum Messen der ultravioletten Strahlen, der mit der Magnetkarte (9) verbunden ist, um das "Schließen des Magnetventils (10) zu steuern, wenn der genannte Sensor (12) ein Signal aussendet, das einer Menge ultravioletter Strahlen entspricht, die unter einem vorbestimmten Schwellenwert liegt,
**dadurch gekennzeichnet, dass** die genannte Magnetkarte (9) Mittel zum Aufsteuern des Magnetventils (10) umfasst, wenn der Sensor (12) eine Menge ultravioletter Strahlen misst, die über dem vorgegebenen Schwellenwert bei einem bestimmten Durchsatz liegt, sowie dadurch, dass die Vorrichtung mit Mitteln zum Anpassen der Menge (15) versehen ist, die auf den vorbestimmten Schwellenwert abgestimmt sind.

2. Vorrichtung nach Anspruch 1, **dadurch gekennzeichnet, dass** das Magnetventil (10) dem Sterilisator (1) bezogen auf die Fließrichtung der Flüssigkeit vorgeschaltet ist.

3. Vorrichtung nach einem der Ansprüche 1 und 2, **dadurch gekennzeichnet, dass** die Mittel zur Abgabe (6) der Flüssigkeit einen Stutzen umfassen, der frei gelassen und ohne Hindernis das Ablaufen von der Austrittsöffnung (4) des Sterilisators (1) bis zum engen Hals (6a) der Abgabemittel (6) ermöglicht.

4. Vorrichtung nach Anspruch 3, **dadurch gekennzeichnet, dass** die Mittel zum Verteilen (6) eine Innenfläche umfassen, die in Kontakt ist mit der Flüssigkeit und aus bioziden, insbesondere silberdotierten und/oder die von der UV-Lampe (2) ausgesandten UV-Strahlen reflektierenden Materialien besteht.

5. Vorrichtung nach einem der Ansprüche 1 bis 4, **dadurch gekennzeichnet, dass** sie Mittel zum beschleunigten Einschalten der UV-Lampe (2) umfasst.

6. Vorrichtung nach Anspruch 5, **dadurch gekennzeichnet, dass** die Magnetkarte (9) an einen Präsenzmelder angeschlossen ist, um ein früheres Einschalten der UV-Lampe zu bewirken, wenn der Präsenzmelder eine Präsenz feststellt.

7. Vorrichtung nach einem der Ansprüche 1 bis 6, **dadurch gekennzeichnet, dass** die Magnetkarte (9) Hilfs-Auslösemittel zum Reinigen des Sterilisators (1), der Ausgabemittel (6) und der Zuführmittel (5) der Flüssigkeit umfasst.

8. Vorrichtung nach einem der Ansprüche 1 bis 7, **dadurch gekennzeichnet, dass**, wenn das Magnetventil (10) geschlossen ist, die Vorrichtung eine bakteriologische Schranke vor und nach dem Sterilisator umfasst, welche bakteriologische Schranke durch das regelmäßige Einschalten der UV-Lampe (2) erzeugt wird.

9. Verfahren zum Schützen einer Flüssigkeit an der Stelle ihrer Benutzung, das eine Vorrichtung mit einem UV-Strahlen-Sterilisator und ein Magnetventil nach einem der Ansprüche 1 bis 8 einsetzt, **gekennzeichnet durch** folgende aufeinanderfolgende Schritte:
- Senden eines ersten Signals an eine Magnetkarte (9) **durch** Aktivieren einer Betätigungseinrichtung (8),
- Steuerung des Einschaltens einer UV-Strahlen-Lampe (2) eines Sterilisators (1), wenn die Magnetkarte (9) das erste Signal empfängt,
- Messen einer von der Lampe (2) ausgesandten Menge ultravioletter Strahlen mittels eines Messfühlers (12), der ein zweites Signal zur Magnetkarte (9) sendet, bis ein vorbestimmter Schwellenwert für eine gegebene Menge überschritten ist,
- Öffnung des Magnetventils (10) beim Empfang eines dritten, von der Magnetkarte (9) ausgesandten Signals, wenn die Karte (9) das zweite Signal empfängt, das den vorbestimmten Schwellenwert überschreitet,
- und Schließen des Magnetventils (10), wenn die von der UV-Lampe ausgestrahlte UV-Strahlenmenge wieder unter den genannten Schwellenwert fällt oder wenn die Betätigungseinrichtung wieder aktiviert wird.

10. Verfahren nach Anspruch 9, **dadurch gekennzeichnet, dass**, wenn die Betätigungseinrichtung erneut aktiviert ist, um das Stoppen der Abgabe von Flüssigkeit anzufordern, das Magnetventil (10) geschlossen wird und die UV-Lampe (2) nach einer Zeit, die von dem vorbestimmten Schwellenwert, von der Leistung der UV-Lampe (2) und vom Volumen des Sterilisators abhängig ist, ausgeschaltet wird.
